# EUROPEAN PATENT APPLICATION

(11) **EP 4 112 084 A1**
(43) Date of publication of application: **04.01.2023**
(21) Application number: 21183270.4
(22) Date of filing: 01.07.2021
(51) Int. Cl.: A61K 49/22, A61K 41/00

(54) **GAS-FILLED MICROBUBBLE AND METHOD FOR FABRICATING A GAS-FILLED MICROBUBBLE**

(71) Applicant: Imec VZW, 3001 Leuven (BE); Universiteit Hasselt, 3500 Hasselt (BE)
(72) Inventor: ETHIRAJAN, Anitha, 3500 Hasselt (BE); JENJOB, Ratchapol, 3001 Leuven (BE)
(74) Representative: Roth, Sebastian

(57) **Abstract**

The disclosure relates to a gas-filled microbubble (10), comprising: a shell (11) encapsulating a gas volume; wherein the shell (11) comprises a gas impermeable molecular layer; wherein the shell (11) is functionalized with a plurality of polymerizable molecules, wherein the polymerizable molecules comprise pentacosadienoic acid, PCDA, derivatives, in particular polyethylene glycol PCDA, PCDA-PEG (13-1, 13-2); wherein the polymerizable molecules are configured to undergo polymerization when being irradiated with UV radiation in a determined wavelength range; and wherein the polymerization of the polymerizable molecules changes physicochemical properties, such as viscoelastic properties, of the microbubble (10).

## Description

### TECHNICAL FIELD

The present disclosure relates to microbubbles for biomedical applications, specifically for use as ultrasonic contrast agents. In particular, the present disclosure relates to a gas-filled microbubble and to a method for fabricating such a gas-filled microbubble.

### BACKGROUND

It is known to use gas-filled microbubbles as ultrasonic contrast agents (UCAs). Such microbubbles are, for example, functionalized with targeting ligands that bind to specific receptors in a patient's body. The microbubbles provide a distinctive acoustic response, which can be read out by an ultrasound imaging device.

For some applications, it would be desirable to generate detectable changes in the acoustic response of microbubbles, e.g. to directly monitor a medical procedure in a patient's body. In principle, such changes in the acoustic response of microbubbles can be caused by a change of size of the microbubbles.

However, it is ambiguous to use changes in size as a parameter to study acoustic property changes of microbubbles, because the stability of the microbubbles is essential, and the microbubbles are prone to change in size when the gas diffuses out. Thus, it is desirable to have microbubbles which can undergo detectable changes, especially in their acoustic response, and are, at the same time, stable in size.

### SUMMARY

Thus, it is an objective to provide an improved gas-filled microbubble, and to provide an improved method for fabricating a gas-filled microbubble. In particular, the above-mentioned disadvantages should be avoided.

The objective is achieved by the embodiments provided in the enclosed independent claims. Advantageous implementations of the embodiments of the invention are further defined in the dependent claims.

According to a first aspect, the present disclosure relates to a gas-filled microbubble, comprising: a shell encapsulating a gas volume; wherein the shell comprises a gas impermeable molecular layer; and wherein the shell is functionalized with a plurality of polymerizable molecules, wherein the polymerizable molecules comprise pentacosadienoic acid (PCDA) derivatives, in particular polyethylene glycol PCDA (PCDA-PEG). The polymerizable molecules are configured to undergo polymerization when being irradiated with UV radiation in a determined wavelength range; wherein the polymerization of the polymerizable molecules changes physicochemical properties, such as viscoelastic properties, of the microbubble.

This achieves the advantage that a microbubble can be provided that is highly stable, radiation sensitive and biocompatible. In particular, the functionalization of the microbubble with the plurality of polymerizable molecules enhances its mechanical stability.

The microbubble can be used as ultrasonic contrast agent (UCA). For instance, the gas-filled microbubble comprises functional groups which allow targeting specific tissues, e.g. tumor tissue. The functional groups can comprise targeting ligands.

The gas-filled microbubble can have a size between 0.5 and 20 µm, preferably between 1 and 10 µm.

The gas, which is encapsulated by the shell of the microbubble, can be perfluorobutane. Alternatively, the gas can also be air or an air perfluorobutane mixture.

The determined wavelength range depends on the polymerizable molecules and is, for instance, between 200 and 280 nm for the PCDA-PEG.

The physicochemical properties of the gas-filled microbubble can be mechanical properties of the microbubble, such as elastic or viscoelastic properties of the microbubble.

The change of the physicochemical properties of the microbubble can be caused by the crosslinking of the polymerizable molecules upon polymerization. In particular, the polymerizable molecules are configured to exhibit a change in their physiochemical and/or viscoelastic properties upon irradiation with the UV radiation, e.g. they become rigid after crosslinking. This change in the physio-chemical/or viscoelastic properties of the polymerizable molecules causes the changes in the physicochemical and/or elastic properties of the microbubble.

In an embodiment, the microbubble is configured to exhibit a change in its acoustic properties due to the change of its physicochemical properties caused by the polymerization of the polymerizable molecules.

In particular, the change in the acoustic properties of the microbubble is caused by a change of the viscoelastic properties of the microbubble upon polymerization of the polymerizable molecules. For instance, the polymerization causes crosslinking of the polymerizable molecules which increases their rigidity, and which, in turn, changes the acoustic properties of the microbubble (e.g, the resonance frequency of the microbubble).

The change in the acoustic properties can comprise a change in the resonance frequency of the microbubble and/or a change in the attenuation of an ultrasound signal by the microbubble. At the same time, the polymerization of said molecules causes no or only a minimal change of the size of the microbubble.

For example, this change in the acoustic properties of the microbubble can be read out with a suitable measurement instrument, e.g. a medical imaging tool, and can be used to generate a 2D or 3D image.

In an embodiment, the PCDA derivatives comprise PEG monosubstituted with PCDA (monoPCDA-PEG) and PEG disubstituted with PCDA (biPCDA-PEG).

By functionalizing the shell with both monoPCDA-PEG and biPCDA-PEG, the stability of the microbubble can be further enhanced. In particular, the microbubble is stable in size before and after polymerization.

In an embodiment, the biPCDA-PEG molecules provide between 60% and 90%, preferably between 70% and 80%, more preferably around 75%, of the total PCDA derivatives.

In particular, the ratio of biPCDA-PEG molecules to monoPCDA-PEG molecules is between 3:2 and 9:1, preferably between 7:3 and 4:1, more preferably around 3:1.

In an embodiment, the gas impermeable molecular layer is formed from a phospholipid layer. In particular, the phospholipid layer can be a monolayer, which is impermeable to the encapsulated gas.

For example, the microbubble shell can comprise 1,2-distearoyl-sn-glycero-3-phosphocholine (DSPC) and/or 1,2-distearoyl-sn-glycero-3-phosphoethanolamine-N-[maleimide(polyethylene glycol)-2000] (DSPE-PEG maleimide) molecules. In particular, the gas impermeable molecular layer is formed from a DSPC layer, which is functionalized with the DSPE-PEG maleimide molecules.

The molar ratios of DSPC : DSPE-PEG maleimide : monoPCDA-PEG : biPCDA-PEG, especially when forming the microbubble, can be: 26 : 1 : 1.3 : 3.2. However, other composition ratios are possible. For example, the proportion of each component of the composition can be increased or decreased by up to 25%.

In an embodiment, the gas-filled microbubble further comprises a plurality of scintillating nanoparticles; wherein said scintillating nanoparticles are configured to convert X-ray radiation into UV radiation in the determined wavelength range.

This achieves the advantage that the microbubbles can be used as UCAs for radiation therapy, in particular for radiation dose monitoring. For instance, the microbubbles can translate an imparted radiation dosage into a modulation of their viscoelastic properties. This can lead to a detectable change in the acoustic repose of the microbubbles upon ultrasound interrogation. Based on said acoustic response after a radiation treatment, a two-dimensional or three-dimensional dose distribution map can be generated.

In an embodiment, the scintillating nanoparticles are arranged to irradiate the polymerizable molecules with the UV radiation in the determined wavelength range, when being irradiated with the X-ray radiation.

In particular, the scintillating nanoparticles carry out an in-situ down-conversion of the X-ray radiation into the UV radiation in the determined wavelength range. In this way, the polymerization the PCDA-PEG molecules can be triggered by the X-ray radiation due to the in-situ down-conversion of said X-ray radiation into UV light in the suitable wavelength range.

In an embedment, the scintillating nanoparticles comprise any one of the following materials: Y₂O₃, CeF₃, LiLuF₄Pr³⁺, LiLuF₄Ce³⁺, LiYF₄Ce³⁺, or LuPO₄Pr³⁺Nd³⁺.

In an embodiment, the scintillating nanoparticles comprise a silicon dioxide, SiO₂, coating with functional groups.

The functional groups can be attached to the SiO₂ surface coating on the nanoparticles. The functional groups may comprise thiol (SH) and/or OH-groups. Furthermore, the functional groups can comprise amines and/or carboxyl groups.

In an embodiment, the scintillating nanoparticles are linked to the shell via linker molecules, in particular 1,2-distearoyl-sn-glycero-3-phosphoethanolamine-N-[maleimide(polyethylene glycol)-2000], DSPE-PEG maleimide.

Alternatively the scintillating nanoparticles can also be linked to the shell via DSPE-PEG carboxylic acid or DSPE-PEG TMS.

According to a second aspect, the present disclosure relates to a method for fabricating a gas-filled microbubble, comprising the steps of:
- forming a shell which encapsulates a gas volume, wherein the shell comprises a gas impermeable molecular layer; and
- functionalizing the shell with a plurality of polymerizable molecules;
wherein the polymerizable molecules comprise pentacosadienoic acid (PCDA) derivatives, in particular polyethylene glycol PCDA (PCDA-PEG); wherein the polymerizable molecules are configured to undergo polymerization when being irradiated with UV radiation in a determined wavelength range; and wherein the polymerization of the polymerizable molecules changes physicochemical properties, such as viscoelastic properties, of the microbubble.

This achieves the advantage that a microbubble can be provided that is highly stable, radiation sensitive and biocompatible. In particular, the functionalization of the microbubble with the plurality of polymerizable molecules enhances its mechanical stability. This microbubble can be used as ultrasonic contrast agent (UCA).

In particular, the microbubble is configured to exhibit a change in its acoustic properties due to the change of its physicochemical properties, especially its viscoelastic properties, caused by the polymerization of the polymerizable molecules.

In an embodiment, the microbubble, in particular the shell of the microbubble, is formed by adding a phospholipid solution and a gas, in particular perfluorobutane, to a reaction volume and ultrasonicating the reaction volume at least once.

In an embodiment, the PCDA derivatives comprise PEG monosubstituted with PCDA (monoPCDA-PEG) and PEG disubstituted with PCDA (biPCDA-PEG).

In an embodiment, the biPCDA-PEG molecules provide between 60% and 90%, preferably between 70% and 80%, more preferably around 75%, of the total PCDA derivatives.

In an embodiment, the gas impermeable molecular layer is formed from a phospholipid layer.

In an embodiment, the method comprises the further step of:
- linking a plurality of scintillating nanoparticles to the microbubble shell;
wherein said scintillating nanoparticles are configured to convert X-ray radiation into UV radiation in the determined wavelength range.

In an embodiment, the scintillating nanoparticles are linked to the shell via linker molecules, in particular 1,2-distearoyl-sn-glycero-3-phosphoethanolamine-N-[maleimide(polyethylene glycol)-2000] (DSPE-PEG maleimide).

For example, the total shell composition of the microbubble is DSPC : DSPE-PEG maleimide : monoPCDA-PEG : biPCDA-PEG with respective molar ratios of 26 : 1 : 1.3 : 3.2.

Alternatively the scintillating nanoparticles can also be linked to the shell via DSPE-PEG carboxylic acid or DSPE-PEG TMS.

In an embedment, the scintillating nanoparticles comprise any one of the following materials: Y₂O₃, CeF₃, LiLuF₄Pr³⁺, LiLuF₄Ce³⁺, LiYF₄Ce³⁺, or LuPO₄Pr³⁺Nd³⁺.

In an embodiment, the scintillating nanoparticles comprise a silicon dioxide, SiO₂, coating with functional groups.

### BRIEF DESCRIPTION OF THE DRAWINGS

Embodiments of the invention will be explained in the followings together with the figures.
- Fig. 1: shows a schematic diagram of a gas-filled microbubble according to an embodiment;
- Fig. 2: shows a schematic diagram of a gas-filled microbubble according to an embodiment;
- Fig. 3: shows a schematic diagram of a down-conversion of X-ray radiation by a scintillating nanoparticle according to an embodiment;
- Figs. 4a-b: show measurements of the size-distribution of gas-filled microbubbles upon polymerization according to an embodiment;
- Figs. 5a-b: show measurements of changes in the optical response of different gas-filled microbubbles upon irradiation with UV and X-ray radiation according to an embodiment;
- Fig. 6: shows schematic diagrams of alternative microbubble designs according to an embodiment;
- Fig. 7: shows steps of a method for fabricating a gas-filled microbubble according to an embodiment;
- Fig. 8: shows an example for fabricating a gas-filled microbubble according to an embodiment; and
- Fig. 9: shows a reaction scheme for the syntheses of monoPCDA-PEG and biPCDA-PEG according to an embodiment.

### DETAILED DESCRIPTION OF EMBODIMENTS

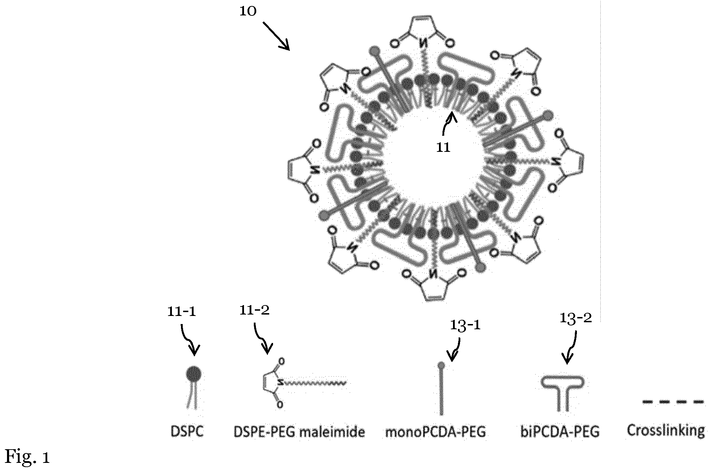

Fig. 1 shows a schematic diagram of a gas-filled microbubble 10 according to an embodiment. In particular, the upper section of Fig. 1 shows a schematic depiction of the microbubble 10 and the bottom section of Fig. 1 shows schematic depictions of its molecular components.

The gas-filled microbubble 10 comprises a shell 11 encapsulating a gas volume, wherein the shell 11 comprises a gas impermeable molecular layer. The shell 11 is functionalized with a plurality of polymerizable molecules, wherein the polymerizable molecules comprise pentacosadienoic acid (PCDA) derivatives, in particular polyethylene glycol PCDA (PCDA-PEG) 13-1, 13-2. The polymerizable molecules are configured to undergo polymerization when being irradiated with UV radiation in a determined wavelength range, wherein the polymerization of the polymerizable molecules changes physicochemical properties of the gas-filled microbubble 10.
The physiochemical properties of the microbubble can be mechanical properties of the microbubble, such as viscoelastic properties of the microbubble.

In particular, the change of the viscoelastic properties of the microbubble 10 due to the polymerization of the polymerizable molecules in turn changes acoustic properties of the microbubble 10. By changing acoustic properties of the microbubble 10, an acoustic response of the microbubble 10 is changed. For example, this change in the acoustic response of the microbubble 10 can be read out with a suitable measurement instrument, e.g. an ultrasound imaging tool, and can be used to generate a two-dimensional or three-dimensional image.

The microbubble 10 can be used as ultrasonic contrast agent (UCA). For instance, the gas-filled microbubble 10 comprise functional groups, e.g. targeting ligands, which allow targeting specific tissues, such as tumor tissue.

In particular, the use of PCDA derivatives, such as 10,12-pentacosadienoic acid, for the polymerizable molecules makes the microbubbles very stable and, at the same time, sensitive to crosslinking by UV irradiation. A change in size of the microbubble 10 after polymerization is thereby mostly prevented.

The PCDA derivatives can comprise monoPCDA-PEG 13-1 and biPCDA-PEG 13-2. In particular, the biPCDA-PEG molecules 13-2 provide between 60% and 90%, preferably between 70% and 80%, more preferably around 75%, of the total PCDA derivatives. By functionalizing the shell with both monoPCDA-PEG 13-1 and biPCDA-PEG 13-2, the stability of the microbubble 10 can be further enhanced. In particular, the microbubble 10 is stable in size before and after polymerization.

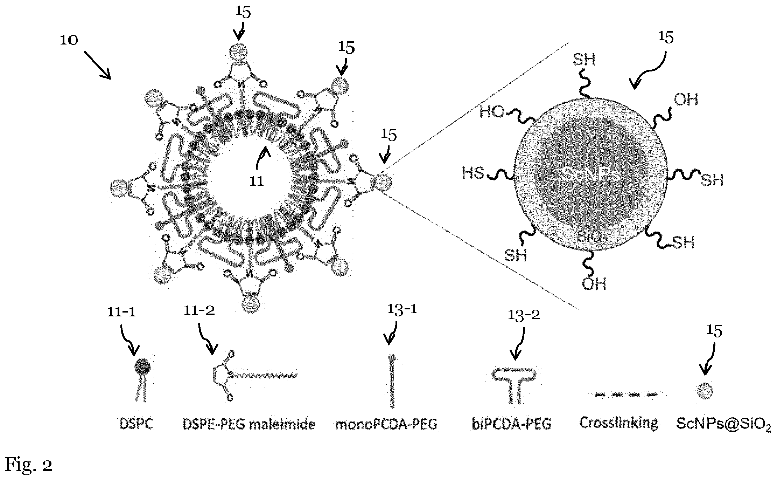

The gas impermeable molecular layer of the shell 11 can be formed from a phospholipid layer. For example, the gas impermeable molecular layer can be formed from 1,2-distearoyl-sn-glycero-3-phosphocholine (DSPC) 11-1, and 1,2-distearoyl-sn-glycero-3-phosphoethanolamine-N-[maleimide(polyethylene glycol)-2000]) (DSPE-PEG maleimide) 11-2. The shell can further be functionalized with PCDA or PCDA derivatives. In particular, the maleimide groups are introduced into the microbubble 10 formulation in order to be a platform for a conjugation with targeting ligands and/or other functional groups or sensitizing agents, e.g. scintillating nanoparticles (see Fig. 2 below).

The molar ratios of DSPC : DSPE-PEG maleimide : monoPCDA-PEG : biPCDA-PEG, especially when forming the microbubble, can be: 26 : 1 : 1.3 : 3.2. However, other composition ratios are possible. For example, the proportion of each component of the composition can be increased or decreased by up to 25%.

The gas, which is encapsulated by the shell 11 of the microbubble 10, can be perfluorobutane.

Fig. 2 shows a schematic diagram of the gas-filled microbubble 10 according to an embodiment.

The gas-filled microbubble in Fig. 2 further comprises a plurality of scintillating nanoparticles 15. The scintillating nanoparticles 15 are configured to convert X-ray radiation into UV radiation in the determined wavelength range.

The scintillating nanoparticles 15 can be linked to the shell 11 of the gas-filled microbubble 10 via the DSPE-PEG maleimides 11-2. Alternatively, also DSPE-PEG carboxylic acid and/or DSPE-PEG TMS can be used as linker molecules for the nanoparticles 15.

The enlarged view of one of the scintillating nanoparticles 15 in Fig. 2 shows that the nanoparticles 15 comprise a core that is surrounded by a SiO₂ coating.

The core of the scintillating nanoparticles 15 can be formed from any one of the following materials: Y₂O₃, CeF₃, LiLuF₄Pr³⁺, LiLuF₄Ce³⁺, LiYF₄Ce³⁺, or LuPO₄Pr³⁺Nd³⁺.

The SiO₂ coating of the nanoparticles 15 can comprise a plurality of functional groups, in particular a mixture of thiol (SH) and OH-groups.

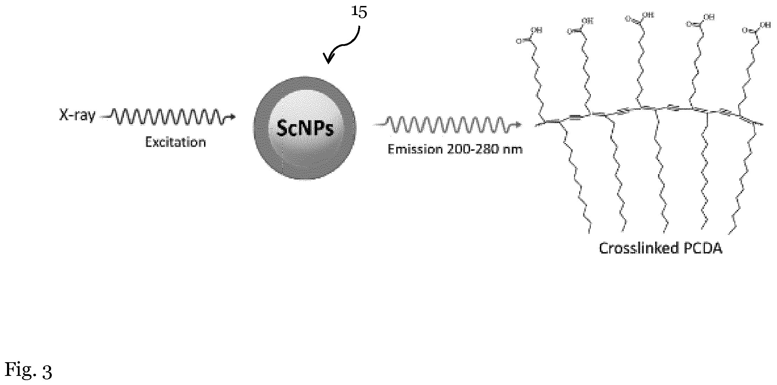

Fig. 3 shows a schematic diagram of a down-conversion of X-ray radiation into UV radiation in the determined wavelength range by one of the scintillating nanoparticles 15 according to an embodiment.

In particular, the scintillating nanoparticles 15 are arranged to irradiate the polymerizable molecules of the microbubble 10 with the UV radiation after said down-conversion, which in-turn induces the polymerization, i.e. crosslinking, of the polymerizable molecules and, thus, may change their acoustic properties.

In other words: the scintillating nanoparticles (ScNPs) 15 can down-convert X-ray radiation into UV radiation, i.e. they can absorb X-ray radiation and re-emit UV radiation. The scintillating nanoparticles 15 are preferably arranged at the vicinity of the polymerizable groups and generate UV radiation in the determined wavelength range for polymerization, e.g. 200-280 nm. This is also advantageous because the UV irradiation necessary for inducing the polymerization generally cannot spread within a body, but the X-ray radiation can.

Thus, by functionalizing the gas-filled microbubble 10 with the scintillating nanoparticles 15, the microbubble 10 can be used as radiation sensitive ultrasound contrast agent that changes its acoustic response upon irradiation with X-ray radiation. The scintillating nanoparticles 15 thereby act as sensitizing agents.

A plurality of such microbubbles 10 can be used as UCAs in a non-invasive in-situ dosimetry system for radiation therapy with the potential of in vivo radiation dose assessment. The microbubbles 10 can be used as dose-sensitive and targeted devices that, for instance, gather in and around tumor tissue and translate an imparted radiation dosage into a modulation of their acoustic response, which can be read out by ultrasound interrogation.

For example, ultrasound read-out technology, e.g. ultrasound imaging and advanced signal processing algorithms, can be used to monitor a radiation dose delivered at a site of interest, e.g. in and around the tumor, by extracting a change in acoustic signature of the UCAs from backscatter data and translate this information into a two-dimensional or three-dimensional dose distribution map (depending on the level of targeting specificity).

Due to the use of PCDA-PEG as polymerizable molecules and, particularly, due to the specific ratio of biPCDA-PEG to monoPCDA-PEG, only certain physicochemical properties (such as rigidity, viscoelastic properties) of the microbubble 10 changes upon X-ray irradiation, while the size of the microbubble 10 stays stable before and after irradiation. This size-stability is important because the echogenic properties are sensitive to size variation.

Thus, a hybrid microbubble concept can be provided, where a microbubble shell property can be changed after irradiation by clinical beams. Thereby, a mechanism for the acoustic property change can be elucidated. The design of the microbubble 10 can include a specific composition of the microbubble shell 11 that comprises a combination of functional molecules/sensitizing agents and a specific design.

Such gas-filled microbubbles 10 with or without the scintillating nanoparticles 15 may also be used for a plurality of different applications, such as in vivo dosimetry, medical imaging, particularly for advanced acoustics read-outs. The gas-filled microbubbles 10 could further be used as theranostic agents, i.e. for therapy and diagnostics. As ultrasound readouts are a commonly used medical imaging tool in the hospitals, the UCAs could be used to directly image diseased tissues during a treatment of said tissue. For example, UV irradiation of cancer cells could be monitored directly via the microbubble UCAs. Alternatively, the down-conversion of X-ray to UV using scintillating nanoparticles 15 at a diseased tissue could be used to damage affected tissues locally. This can lead to the synergistic effect that the X-ray dose can be reduced which might improve patient compliance. On the other hand, the cost of the therapy could be reduced. Additionally, UV cleavable drug conjugates, which could be attached to the microbubble 10 or the nanoparticles 15, could be used to deliver different types of drugs at an affected site.

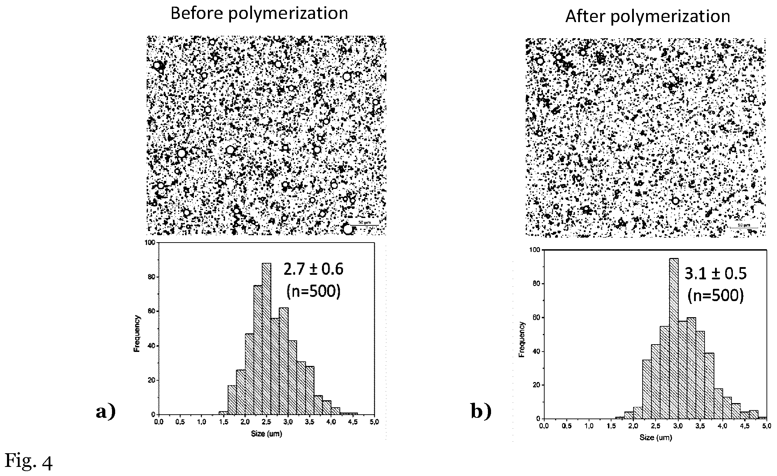

Figs. 4a-b show measurements of the size-distributions of gas-filled microbubbles 10 upon polymerization according to an embodiment.

Fig. 4a shows a microscopic image (top) of a microbubble solution and a histogram of the size distribution of 500 microbubbles (bottom) in the solution, before a polymerization of the microbubbles. The microbubbles comprise a monoPCDA-PEG:biPCDA-PEG ratio of 25:75.

Fig. 4b shows a microscopic image and a size distribution of the same microbubble solution after polymerization via UV irradiation at 254 nm for 5 min.

The comparison between the microscope images and histogram data in Figs. 4a and 4b shows that the microbubbles exhibit only a small change upon polymerization. The observed average change in size of the microbubbles from 2.7 µm to 3.1 µm is within the measurement error and, thus, neglectable.

Microbubbles with different ratios of monoPCDA-PEG:biPCDA-PEG; e.g. 50:50, exhibit much larger changes in size, while the change in size with microbubbles which only comprise biPCDA-PEG is the largest.

The high stability of gas-filled microbubbles with a monoPCDA-PEG:biPCDA-PEG ratio of 25:75 can also be shown by other measurements,

In addition, the scintillating nanoparticles 15 may further improve the stability of the microbubbles. This can be attributed to the nanoparticles 15 attached to the surface of the microbubble 10 acting as an additional barrier for gas diffusion. In particular, it can be shown that microbubbles with nanoparticles are more stable than microbubbles without the nanoparticles before and after irradiation.

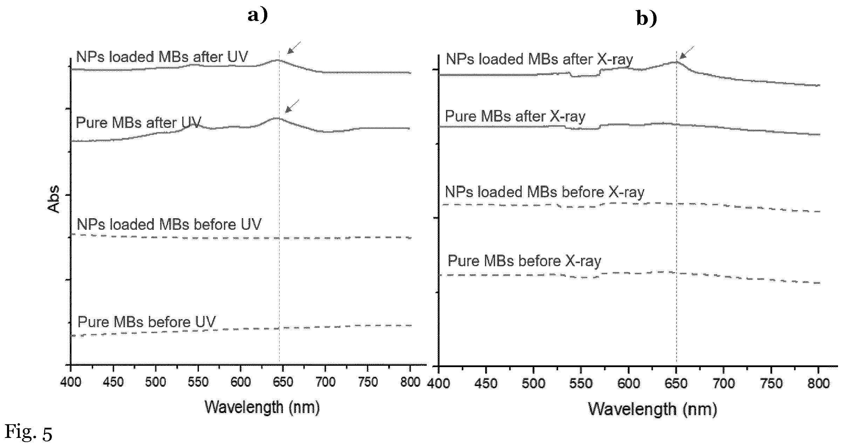

Figs. 5a-b show measurements of changes in the optical response of different gas-filled microbubbles 10 upon irradiation with UV and X-ray radiation according to an embodiment.

In particular, Figs. 5a and 5b show UV-Vis spectra of solutions of microbubbles 10 with and without scintillating nanoparticles 15. The recorded spectra in Fig. 5a and 5b are shifted on the y-axis for better comparison.

The microbubbles 10 used for these optical characterization measurements comprise DSPC, DSPE-PEG maleimide, as well as mono & biPCDA-PEG.

Fig. 5a shows UV-Vis spectra of both microbubbles solutions prior to a UV irradiation (dashed lines) and after a UV irradiation (bold lines). The UV irradiation was carried out at the determined wavelength range for polymerization of the polymerizable molecules. As can be seen, the UV-vis spectra of the microbubbles without scintillating nanoparticles 15 (Pure MBs) and the microbubbles with scintillating nanoparticles (NPs loaded MBs) essentially exhibit the same change after UV irradiation. Both spectra develop a peak at ca. 650 nm after irradiation (marked by an arrow).

Fig. 5b shows UV-Vis spectra of both microbubbles solutions prior to an X-ray irradiation (dashed lines) and after an X-ray irradiation (bold lines). The comparison of the two spectra after the X-ray irradiation shows that only the microbubbles 10 loaded with nanoparticles 15 exhibit a strong change in the spectrum and develop the peak at 650 nm. Essentially no change in the spectra after X-ray irradiation occurs for microbubbles 10 without nanoparticles 15.

These results show that the scintillating nanoparticles 15 are capable to down convert X-ray radiation into UV radiation to cause a polymerization of the polymerizable molecules of microbubbles 10. Thus, these gas-filled microbubbles 10 are suitable for use as radiation sensitive UCAs.

Furthermore, it was observed that the acoustic attenuation of microbubbles 10 with scintillating nanoparticles 15 changes upon irradiation by clinical megavoltage beams, whereas the microbubble without the nanoparticles did not show characteristic changes in acoustic attenuations.

The polymerization of microbubbles with the scintillating nanoparticles 15 can also be observed by a peak in the optical spectra and a visual color change of a microbubble solution after X-ray irradiation, while the microbubbles without scintillating nanoparticles 15 show no such visible response.

For example, a solution of microbubbles based on DSPC/DSPE-PEG maleimide/mono&biPCDA-PEG loaded with scintillating nanoparticles 15 with a SiO₂ coating (ScNPs@SiO₂) has a whitish or grayish color prior to X-ray irradiation. After X-ray irradiation, the solution takes on a blueish color.

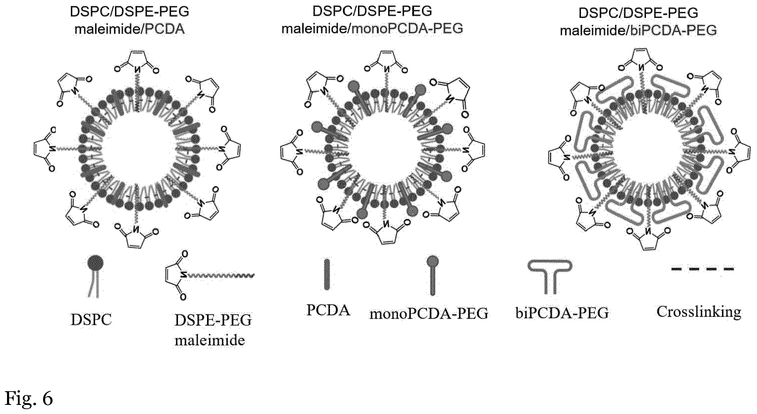

Fig. 6 shows schematic diagrams of alternative gas-filled microbubble 10 designs according to an embodiment.

In particular, Fig. 6 shows a microbubble 10 formed from DSPC, DSPE-PEG maleimide and pentacosadienoic acid (PCDA) (left), a microbubble 10 formed from DSPC, DSPE-PEG maleimide and monoPCDA-PEG (middle) and a microbubble 10 formed from DSPC, DSPE-PEG maleimide and biPCDA-PEG (right).

However, these microbubble 10 designs are less stable in size than the microbubbles 10 shown in Figs. 1 and 2 that comprise a mixture of biPCDA-PEG and monoPCDA-PEG, preferably at a ratio of roughly 3:1.

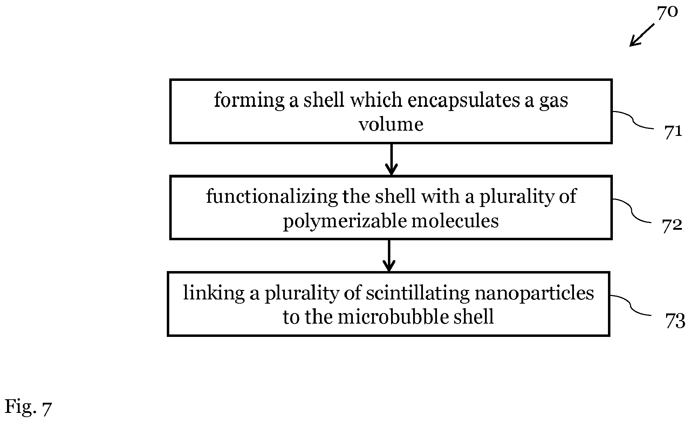

Fig. 7 steps of a method 70 for fabricating the gas-filled microbubble 10 according to an embodiment. In particular, the method 70 can be used to fabricate a plurality of gas-filled microbubbles 10 at the same time, e.g. a solution containing a plurality of gas-filled microbubbles 10.

The method comprises 70:
- forming 71 the shell 11 which encapsulates a gas volume, wherein the shell 11 comprises a gas impermeable molecular layer; and
- functionalizing 72 the shell 11 with a plurality of polymerizable molecules.

Thereby, the polymerizable molecules comprise PCDA derivatives, in particular PCDA-PEG 13-1, 13-2. The polymerizable molecules are configured to undergo polymerization when being irradiated with UV radiation in the determined wavelength range, wherein the polymerization of the polymerizable molecules changes physicochemical properties, such as viscoelastic properties, of the gas-filled microbubble 10.

The change in the physicochemical and/or viscoelastic properties upon polymerization of the polymerizable molecules may cause a change in the acoustic properties of the microbubble 10.

The method 70 can comprise the further step of:
- linking 73 a plurality of scintillating nanoparticles 15 to the shell 11, wherein the scintillating nanoparticles 15 are configured to convert X-ray radiation into UV radiation in the determined wavelength range.
In this way, gas-filled microbubbles can be generated that can change their physicochemical properties and, particularly, their acoustic properties upon X-ray irradiation.

The PCDA derivatives can comprise of monoPCDA-PEG and biPCDA-PEG.

The scintillating nanoparticles 15 can be linked to the shell via linker molecules, such as DSPE-PEG maleimide.

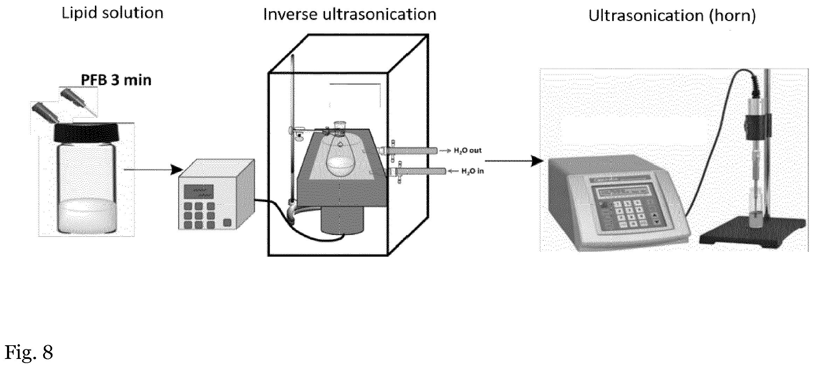

Fig. 8 shows an example for fabricating gas-filled microbubbles 10 according to an embodiment. In particular, Fig. 8 shows an example of how the method steps 71 and 72 of the method 70 shown in Fig. 7 can be carried out.

The method shown in Fig. 8 can be used to process a gas-filled microbubble 10 with a shell 11 that is functionalized with monoPCDA-PEG 13-1 and biPCDA-PEG 13-2 at a fixed ratio. The method comprises dissolving DSPC 11-1, biPCDA-PEG 13-2, monoPCDA-PEG 13-1 and DSPE-PEG maleimide 11-2 in, for instance, in chloroform, followed by drying until the ingredients form a film. After chloroform removal, a phosphate-buffered saline (PBS) solution is added.

In a subsequent step, the phospholipid solution is fluxed by perfluorobutane (PFB) (Fig. 8, left). Subsequently, an inverse ultrasonication is used to create emulsion droplets of the lipid solution (Fig. 8, middle). The microbubbles (MBs) can be formed using an ultrasonication horn (Fig. 8, right).

In particular, during the inverse respectively indirect ultrasonication (Fig. 8, middle), the vial with the phospholipid solution can be placed in a small cup with water and energy is provided from outside of the vial. In case of the ultrasonication with a horn/tip (Fig. 8, right), the tip can be placed in the phospholipid solution directly. With the inverse ultrasonication, only perfluorobutane (PFB) gas is present inside the closed vial. In contrast, when using the ultrasonication horn respectively tip, the sample is exposed to air. By first generating larger microbubbles 10 via indirect ultrasonication, it can be avoided that air is enclosed in the microbubbles 10 to a large extent

The fabricated microbubbles 10 can be subsequently loaded with the scintillating nanoparticles 15. The nanoparticles 15 can be added into a microbubble solution of, e.g., via a syringe. Any excess nanoparticles 15 can be removed, e.g. by a gravimetric analysis, and the loading content of the nanoparticles 15 on the microbubbles 10 can be calculated.

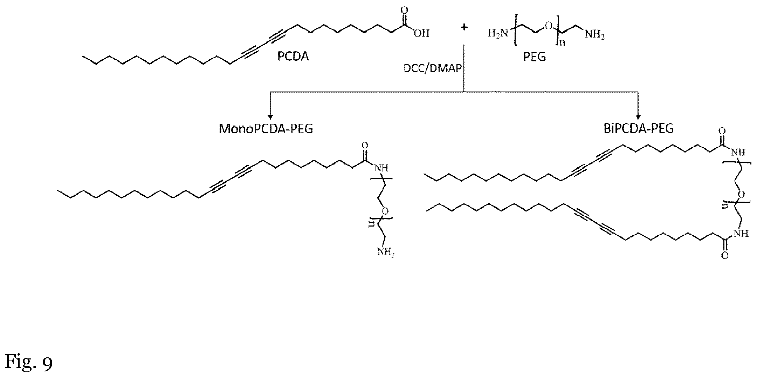

Fig. 9 shows a reaction scheme for the syntheses of monoPCDA-PEG 13-1 and biPCDA-PEG 13-2 according to an embodiment.

## Claims

1. A gas-filled microbubble (10), comprising:
a shell (11) encapsulating a gas volume;
wherein the shell (11) comprises a gas impermeable molecular layer;
wherein the shell (11) is functionalized with a plurality of polymerizable molecules,
wherein the polymerizable molecules comprise pentacosadienoic acid, PCDA, derivatives, in particular polyethylene glycol PCDA, PCDA-PEG (13-1, 13-2);
wherein the polymerizable molecules are configured to undergo polymerization when being irradiated with UV radiation in a determined wavelength range; and
wherein the polymerization of the polymerizable molecules changes physicochemical properties, such as viscoelastic properties, of the microbubble (10).

2. The gas-filled microbubble (10) of claim 1,
wherein the microbubble (10) is configured to exhibit a change in its acoustic properties due to the change of its physicochemical properties caused by the polymerization of the polymerizable molecules.

3. The gas-filled microbubble (10) of claims 1 or 2,
wherein the PCDA derivatives comprise PEG monosubstituted with PCDA, monoPCDA-PEG (13-1), and PEG disubstituted with PCDA, biPCDA-PEG (13-2).

4. The gas-filled microbubble (10) of claim 3,
wherein biPCDA-PEG molecules provide between 60% and 90%, preferably between 70% and 80%, more preferably around 75%, of the total PCDA derivatives.

5. The gas-filled microbubble (10) of any one of the preceding claims,
wherein the gas impermeable molecular layer is formed from a phospholipid layer.

6. The gas-filled microbubble (10) of any one of the preceding claims, further comprising:
a plurality of scintillating nanoparticles (15);
wherein said scintillating nanoparticles (15) are configured to convert X-ray radiation into UV radiation in the determined wavelength range.

7. The gas-filled microbubble (10) of claim 6,
wherein the scintillating nanoparticles (15) are arranged to irradiate the polymerizable molecules with the UV radiation in the determined wavelength range, when being irradiated with the X-ray radiation.

8. The gas-filled microbubble (10) of claim 6 or 7,
wherein the scintillating nanoparticles (15) comprise any one of the following materials: Y₂O₃, CeF₃, LiLuF₄Pr³⁺, LiLuF₄Ce³⁺, LiYF₄Ce³⁺, or LuPO₄Pr³⁺Nd³⁺.

9. The gas-filled microbubble (10) of any one of claims 6 to 8,
wherein the scintillating nanoparticles comprise a silicon dioxide, SiO₂, coating with functional groups.

10. The gas-filled microbubble (10) of any one of claims 6 to 9,
wherein the scintillating nanoparticles (15) are linked to the shell (11) via linker molecules, in particular 1,2-distearoyl-sn-glycero-3-phosphoethanolamine-N-[maleimide(polyethylene glycol)-2000], DSPE-PEG maleimide (11-2).

11. A method (70) for fabricating a gas-filled microbubble (10), comprising the steps of:
- forming (71) a shell (11) which encapsulates a gas volume, wherein the shell (11) comprises a gas impermeable molecular layer; and
- functionalizing (72) the shell (11) with a plurality of polymerizable molecules; wherein the polymerizable molecules comprise pentacosadienoic acid, PCDA, derivatives, in particular polyethylene glycol PCDA, PCDA-PEG (13-1, 13-2); wherein the polymerizable molecules are configured to undergo polymerization when being irradiated with UV radiation in a determined wavelength range; and wherein the polymerization of the polymerizable molecules changes physicochemical properties, such as viscoelastic properties, of the microbubble (10).

12. The method (70) of claim 11,
wherein the microbubble (10), in particular the shell (11) of the microbubble (10), is formed by adding a phospholipid solution and a gas, in particular perfluorobutane, to a reaction volume and ultrasonicating the reaction volume at least once.

13. The method (70) of claim 11 or 12,
wherein the PCDA derivatives comprise PEG monosubstituted with PCDA, monoPCDA-PEG (13-1), and PEG disubstituted with PCDA, biPCDA-PEG (13-2).

14. The method (70) of any one of claims 11 to 13, further comprising the step:
- linking (73) a plurality of scintillating nanoparticles (15) to the microbubble (10) shell (11);
wherein said scintillating nanoparticles (15) are configured to convert X-ray radiation into UV radiation in the determined wavelength range.

15. The method (70) of claim 14,
wherein the scintillating nanoparticles (15) are linked to the shell (11) via linker molecules, in particular 1,2-distearoyl-sn-glycero-3-phosphoethanolamine-N-[maleimide(polyethylene glycol)-2000], DSPE-PEG maleimide (11-2).
